# EUROPEAN PATENT APPLICATION

(11) **EP 1 293 226 A1**
(43) Date of publication of application: **19.03.2003**
(21) Application number: 02256188.0
(22) Date of filing: 06.09.2002
(51) Int. Cl.: A61M 15/00

(54) **Disposable personal aromatherapy mask kit**

(30) Priority: 08.09.2001 US 318587 P
(71) Applicant: Dyer, Sally E., Hilton Head Island, South Carolina 29926 (US)
(72) Inventor: Dyer, Sally E., Hilton Head Island, South Carolina 29926 (US)
(74) Representative: Hedley, Nicholas James Matthew

(57) **Abstract**

An aromatherapy inhalation kit providing portable, disposable and immediate therapy. The kit comprises a vial (14) having a capacity of about 2 ml. pre-filled with one application of pre-blended, predetermined quantity and diluted essential oil, a cone-type mask of paper fiber (10) to which the oil blend is applied; and a heavy weight paper folder (22) to hold the mask and vial in the protective folder. At use, the contents of the vial (14) are dispersed onto the outside surface of the mask (10). The mask is then worn over the user's nose and mouth and the user inhales the vapors for twenty to thirty minutes to enhance the user's mood and/or condition. The kit is small enough to carry in an automobile glove compartment, a backpack, a purse or a pocket. Since its administration does not rely upon electrical power, it is convenient to use at any time or in any location.

## Description

### Background of the Invention

The present invention relates to aromatherapy, and, more particularly, to an improved method of the administration of diluted essential oils to a person through the process of inhalation, and to a kit that can be used to carry out the administration of aromatherapy.

Aromatherapy is therapy using essential oils in a variety of ways and for a number of purposes. Essential oils are the volatile, organic constituents of fragrant plant matter, and they contribute to both flavor and fragrance. When distilled for plants, the essential oils become concentrated 100 fold or more, so certain properties become very pronounced in the oils.

Essential oils are obtained from varying parts of different aromatic plants, including leaves, flowers seeds and roots. The oils produce certain mood-enhancing results and serve to rectify and/or change an existing condition in an individual. The most widely used method of obtaining essential oils from fragrant plants is by steam distillation. This process involves vaporizing into steam the essential oils present in the plant (which may be fresh or dried) and then cooling the vapor, which results in condensation. The final product is a complex mixture of odiferous compounds, called an essential oil.

In aromatherapy, the administration of essential oils to the body is accomplished in three principal manners: dermal absorption, inhalation and oral ingestion. The most commonly used route, in both therapy and retail products, is dermal application through the use of massage oils applied directly to the skin or through the use of various beauty products such as soaps, creams, body sprays and the like. The method of application uses the highest dilution rate, so the effects of aromatherapy are minimized. The least common route is oral ingestion mainly due to the fact that doses of essential oils are greatly increased, and much care must be exercised in their administration. The median method involves inhalation which is accomplished by an individual inhaling the vapors of an essential oil.

The most common mechanism for the dispersal of essential oils through inhalation is a mechanical diffuser. The mechanical dispersal distributes vapors to an entire room, or groups of rooms, and presents a clear problem to any person within the range of these vapors who may not wish to be exposed to such vapors. In addition, the use of mechanical diffusers is dependent upon an external electrical power source, or alternatively, on internal battery power. This power dependence greatly decreases the number of opportune locations and situations for aromatherapy administration.

In addition to the problem created by lack of a power source vis-a-vis a mechanical diffuser, it is believed that the majority of individuals who would possibly desire the benefits of aromatherapy are not knowledgeable in either the specificity of the correct oils to use in a given situation, nor in their safe dilution ranges.

In general, no form of inhalation of essential oils for aromatherapy purposes is known to have used a combination of items in a kit comprised of a disposable mask, with a pre-mixed and pre-measured essential oil blend in a one-dose disposable vial.

It would therefore be advantageous to have available, a convenient assemblage of components, in a kit form, that could be supplied to a user and which contains all of the necessary items to conduct inhalation aromatherapy by the user and without the need to dilute or otherwise determine the concentration of a particular essential oil, no matter what that particular oil is and also which can be completely portable, that is, a kit that is readily carried on the person and which does not require any external or internal source of electrical power in order to fully realize the beneficial effects of the therapy.

### Summary of the Invention

The present invention provides an improved kit for the administration of diluted essential oils for aromatherapy through inhalation and which consists of a cone-type disposable mask of paper composition, a glass or plastic vial (of approximately 2 ml. capacity) of premixed, pre-measured essential oils, safely diluted in a vegetable based "carrier oil", a protective heavy weight paper folder into which the oil vial is placed for protection and a clear plastic bag which conveniently contains the components of the kit, i.e. the disposable mask and the folder protecting the vial.

Accordingly, the user can be provided with a variety of personal aromatherapy inhalant treatments in a handy kit which can be used at any time and location a user finds convenient and useful, and which further provides a new aromatherapy inhalation system of pre-mixed and pre-measured diluted essential oils in a safe single dose unit which is administered to a disposable mask worn over the user's mouth and nose.

As a feature of the present aromatherapy kit, therefore, the user can follow instructions that can also be provided within the aforedescribed kit itself and have available, all of the items necessary as well as the instruction of use to carry out inhalation aromatherapy without the need for any addition items.

Other features of the present aromatherapy kit will become apparent in light of the following detailed description of a preferred embodiment thereof and as illustrated in the accompanying drawings.

### Brief Description of the Drawings

FIG. 1 is a front perspective view of the disposable, cone-type mask of the present invention;
FIG 2 is perspective view of a glass vial containing the essential oil of the present invention;
FIG. 3 is a perspective view of a protective folder of the present invention;
FIG. 4 is a front view of the vial of Fig. 2 positioned within the protective folder of Fig. 3;
FIG. 5 is a front view of a clear plastic bag that can be used to enclose the mask of Fig. 2 with the vial of Fig. 2 contained within the protective folder as shown in Fig. 4.

### Detailed Description of the Invention

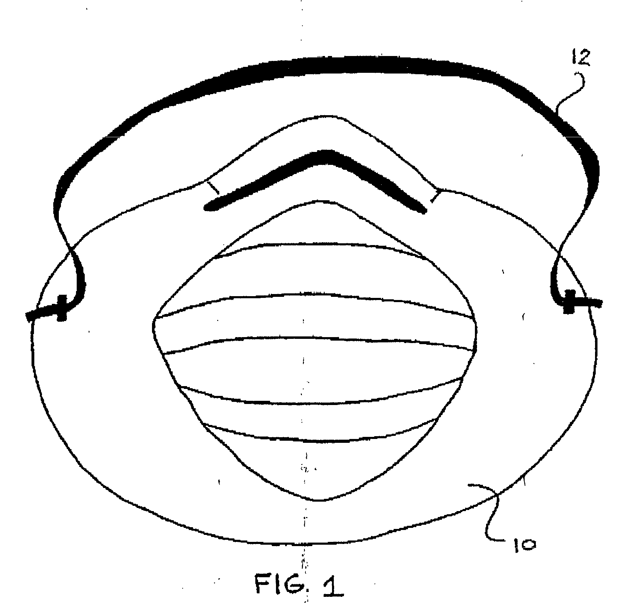

Referring now to FIG. 1, there is shown a front, perspective view of a disposable cone-type mask 10 that can be used with the present invention. As can be seen, the disposable mask 10 is of the type that can be inexpensive so as to be disposable when provided with the present kit and which can be comprised of a cellulose material, such as a paper composition. The disposable mask 10 is dimensioned so as to fit comfortably over the nose and mouth of the user and is sufficiently porous such that the essential oil, having a predetermined concentration and quantity, can simply be poured over the exterior of the disposable mask 10 and the vapors inhaled by the user.

As is conventional, the disposable mask 10 includes an elastic strap 12 that can be fitted around the head of the user to maintain the mask 10 in the proper desired position on the user's face and to allow the user to perform other functions, to move about in a normal manner or, alternatively, to simply comfortably recline as the effects of the essential oil are promoted.

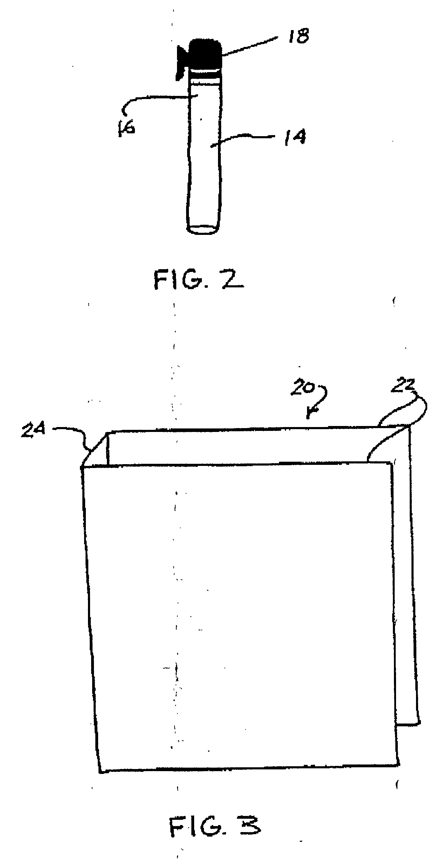

Next, in Fig. 2 there is shown a perspective view of a vial 14 containing the liquid 16 that is comprised of an essential oil and which may be diluted by the use or addition of a diluent such as a vegetable based "carrier " oil, it being of importance that the liquid 16 within the vial 14 has been pre-measured as to its quantity as well as to the concentration of the essential oil within the liquid 16 or other diluent material. A stopper 18, preferably of plastic, can be used to seal the vial 14 after the proper concentration and quantity of liquid 16 containing the essential oil has been introduced into the vial 14 such that the vial 14 can be shipped in the normal channels of trade and the liquid 16 safely remain contained within the vial 14.

The actual concentration of the essential oil and its carrier diluent can, of course, vary among the different essential oils, however, it is preferred that the quantity be in an amount such that the liquid 14 can be fully utilized at a single dosage or therapy session, and such therapy session, may be, generally, from twenty to thirty minutes in length. In the preferred embodiment, the quantity of the liquid 16 is in an amount of about 2 ml. in volume.

Turning next to Fig. 3, there is shown, a perspective view of a protective envelope, or folder 20 that can be used to protect the vial 14 (Fig. 2) during shipment, storage, or transportation to or by the user. In Fig. 3, the folder 20 is formed with a pair of flaps 22 that extend outwardly, in a fairly uniform manner from a spine 24 much in the likeness of a notebook and it is preferred that the folder 20 also be comprised of an inexpensive but protective material such as a heavy-weight paper material, including cardboard, however, other materials can be used such as a plastic foam material that also can be folded as shown in Figure 3.

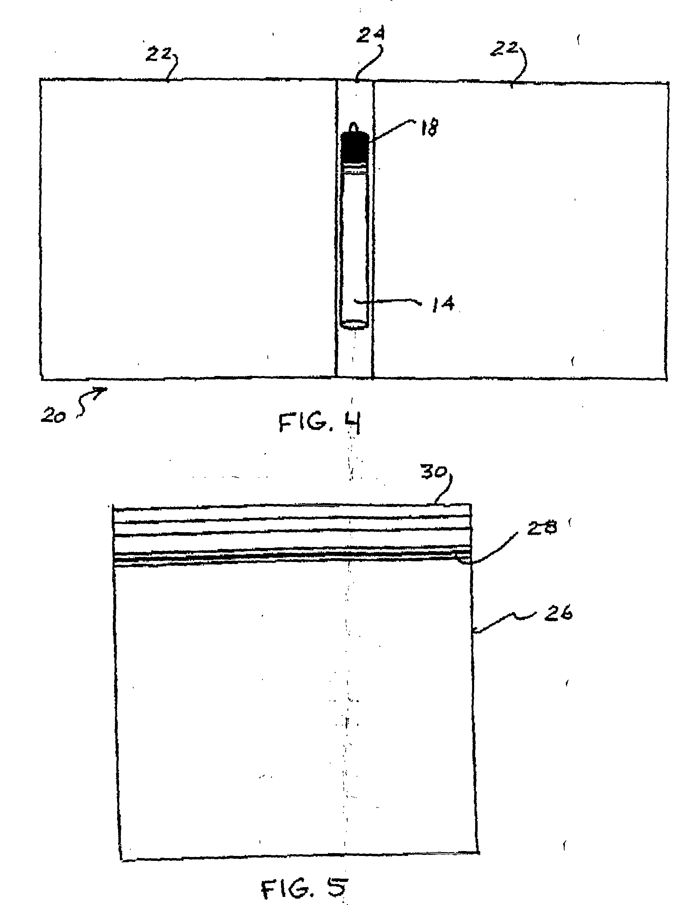

In Fig. 4, there can be seen a front, open view of the protective folder 20 with the vial 14 positioned therein in a protected environment and, as can be seen, the vial 20 rests against the spine 24 of the protective folder 20. As such, the flaps 22 of the protective folder 20 can each be rotated inwardly so as to wrap around the vial 20 and retain the vial 20 therebetween such that the vial 14 is protected by means of the protective folder 20 from breakage that may occur as a result of handling, storage, or simply from being carried around by the user in anticipation of a future use.

As indicated, the size of the present inventive kit and the vial 14 within the protective folder 20 is such that the kit can be readily carried by the user in a purse, pocket, glove compartment of an automobile or the like since the overall kit is sufficiently small to be convenient for the user to take anywhere and still have the necessary items to carry out the process of aromatherapy at any location.

Finally, turning to Fig. 5, there is shown a front view of a carrying container and, as shown the container may be a clear, plastic bag 26 that can protectively carry all of the aforedescribed components, that is, the plastic bag 26 can carry the protective mask 10 (Fig. 1), the vial 14 contained within the protective folder 20 so that the user has a convenient means of carrying all of the components of the kit of the present invention. As indicated, it is preferred that the carrying container be a plastic bag 26 that is transparent, however, other bags or conveyances may be used, it being preferable that the particular container be tightly sealed and, in the embodiment of Fig. 5, that seal may be carried out by the use of a conventional zip-lock 28 that opens and closes the open side 30 of the plastic bag 26.

Accordingly, referring again to Figs 1-5, with the components of the kit of the present invention now identified and described, the method of using those components can now also be outlined. In operation, one uses the aromatherapy inhalation system by first removing the mask 10 from the clear, plastic bag 26 and then by removing the clear plastic or glass vial 14 containing the essential oil that has been blended, diluted or the like from the protective folder 20. The vial 14 is unstopped by removing the plug 18 and the contents of the essential oil that has been provided having the correct concentration and quantity is poured onto the outside surface of the disposable mask 10 as near to the center of the disposable mask 10 as possible.

The disposable mask 10, now having the essential oil seeping through the paper material, is then donned by the user so as to cover the nose and mouth of the user with the elastic strap 12 encircling the back of the head of the user to maintain the disposable mask 10 in the proper position. The user can now inhale the vapors until the desirable result is obtained by the user but, in general, not to exceed a period of between about twenty to thirty minutes.

The particular method of aromatherapy administration through inhalation is accomplished at close range and exclusively for the benefit of the treated individual. The user of the present inhalation aromatherapy system does not have to measure, mix or guess the proportions of the dilution of the essential oils because the proper one-dose application is contained in the enclosed vial 14 that is provided as a component of the present aromatherapy kit. In addition, the use is personal and the aroma from the essential oils is not carried over to other persons in the same room or in the general vicinity of the actual user of the kit.

The most preferred embodiment of the present invention is with the disposable mask using an oil blend under the trademark REVIVE and with the device, that particular essential oil is used to assuage the sleepiness experienced by drivers of personal motor vehicles and trucks when they are on the road for extended periods of time. The aromatherapy kit can be placed in a small storage area of the vehicle, for example, the glove compartment or side-door compartment, ready for use, if and when needed by the driver. The essential oil in the REVIVE mask can quickly and efficiently combat sleepiness through twenty to thirty minutes of inhalation time, thereby enabling the driver to gain alertness when feeling sleepy at the wheel of the vehicle.

Other aromatherapy kits are embodied by, but not necessarily limited to, a mask kit supplied under the trademark RELAX that induces calm, a mask kit supplied under the trademark, REFRESH that blocks foul odors, a mask kit under the trademark RELIEVE that relieves nasal congestion and a mask kit under the trademark REJUVINATE that encourages a romantic mood.

In all, while the foregoing examples are specific, they should not be construed as limitations on the scope of the present invention nor on the myriad of essential oil combinations which are possible to assembly in the kit form. The essence of the invention is in the providing of all of the necessary components or elements for carrying out aromatherapy by the inclusion of a disposable mask, a disposable glass or plastic vial having a volume capacity of about 2 ml. and containing a pre-blended and pre-determined quantity of diluted essential oil, a protective heavy-weight paper folder to hold and protect the vial and a plastic bag having a zip-lock closure to hold the mask and the protective folder containing the vile therein.

It will be understood that the scope of the invention is not limited to the particular embodiment disclosed herein, by way of example, but only by the scope of the appended claims.

## Claims

1. An aromatherapy kit for administering an essential oil to a user, said aromatherapy kit comprising:
a cone-type, disposable face mask,
a vial containing a pre-mixed, predetermined amount, e.g. about 2 ml, of a liquid having an essential oil,
a protective folder for protecting the vial, and
a container for holding the mask and the folder containing the vial.

2. An aromatherapy kit as defined in claim 1 wherein said vile contains the essential oil diluted in a quantity of a carrier oil, which is preferably a vegetable oil.

3. An aromatherapy kit as defined in claim 1 wherein the protective folder comprises a cellulose sheet, folded over to form a pocket and wherein said vial is positioned within the pocket, which cellulose sheet is preferably a heavy-weight paper envelope and said pocket is preferably formed by folding said protective folder to create a spine having two generally parallel flaps extending outwardly from said spine.

4. An aromatherapy kit as defined in claim 1 wherein said container is a flexible transparent plastic bag having a closure to open and close the bag, e.g a zip lock closure.

5. A non-therapeutic method of providing aromatherapy for a user, said method comprising the steps of:
providing a kit comprising a container, which preferably comprising a flexible, transparent plastic material, the container having contained therein, a disposable mask and a vial containing a predetermined quantity, e.g. about 2 ml, of a liquid with an essential oil at a predetermined concentration,
removing the disposable mask for the container,
removing the vial from the container,
opening the vial and pouring the contents of the vial onto the disposable mask,
placing the disposable mask over the nose and mouth of a user and inhaling the vapors provided for the disposable mask to achieve the desired effects from inhalation aromatherapy.

6. A method of providing aromatherapy to a user as defined in claim 5 wherein said step of providing a kit comprises providing a kit having contained therein a vial enclosed in a protective folder that is preferably made of a heavy paper material and that is preferably folded about the vial to form a protective pocket around the vial.

7. An aromatherapy kit for administering aromatherapy to a user, said aromatherapy kit comprising a plurality of components encased within a container and comprising:
a cone-type, disposable face mask,
a vial containing a pre-mixed, predetermined amount, e.g. about 2 ml, of liquid containing an essential oil, and
a protective envelope for protecting the vial.

8. An aromatherapy kit as defined in claim 7 wherein said protective envelope comprises a heavy paper wrapper that at least partially encloses said via, which protective envelope preferably comprises a spine having two flaps extending outwardly from said spine and said vial is protectively located between said flaps and in close proximity to said spine.

9. An aromatherapy kit as defined in claim 7 wherein said liquid comprises an essential oil diluted into a predetermined concentration in a carrier oil.

10. An aromatherapy kit as defined in claim 7 wherein said vial has a removable stopper to contain the liquid therein, which stopper is preferably comprised of a plastic material.
